# EUROPÄISCHE PATENTSCHRIFT

(11) **EP 1 691 694 B1**
(45) Veröffentlichungstag und Bekanntmachung des Hinweises auf die Patenterteilung: **05.09.2007**
(21) Anmeldenummer: 04764940.5
(22) Anmeldetag: 08.09.2004
(51) Int. Cl.: A61B 17/32, A61B 17/22, A61B 17/34

(54) **MEDIZINISCHES INSTRUMENT ZUM SCHNEIDEN VON BIOLOGISCHEM UND INSBESONDERE MENSCHLICHEM GEWEBE**
MEDICAL INSTRUMENT FOR CUTTING BIOLOGICAL AND IN PARTICULAR HUMAN TISSUE
INSTRUMENT MEDICAL SERVANT A COUPER DES TISSUS BIOLOGIQUES, EN PARTICULIER DES TISSUS HUMAINS

(30) Priorität: 11.12.2003 DE 10358279
(43) Veröffentlichungstag der Anmeldung: 23.08.2006
(60) Teilanmeldung: 06004890.7
(73) Patentinhaber: Karl Storz GmbH & Co. KG, 78532 Tuttlingen (DE)
(72) Erfinder: SEEH, Daniel, 78194 Immendingen (DE)
(74) Vertreter: Hofmeister, Frank
(86) Internationale Anmeldenummer: PCT/EP2004/009997
(87) Internationale Veröffentlichungsnummer: WO 2005/060842

(56) Entgegenhaltungen:
- EP-A- 0 769 278
- DE-C1- 4 306 205
- US-A- 5 123 904
- US-A- 5 618 296
- US-B1- 6 439 541
- US-B1- 6 638 238

## Beschreibung

Die Erfindung betrifft ein medizinisches Instrument zum Schneiden von biologischem und insbesondere menschlichem Gewebe mit einem über einen Motor um seine Längsachse drehbaren hohlen Schneidrohr, an dessen distalem Ende mindestens eine Schneide angeordnet ist, sowie mit einer Handhabe, in der das Schneidrohr führend gelagert ist, wobei der Motor als auf dem Schneidrohr angeordneter Hohlwellenmotor ausgebildet ist.

Derartige, Morcellatoren genannte medizinische Instrumente werden bei endoskopischen Eingriffen verwendet, die die Entfernung großer Gewebeanteile notwendig machen. Diese Morcellatoren bestehen aus einem angetriebenen Schneidrohr, das direkt in den Körper bzw. eine natürliche oder künstliche Körperhöhle eingeführt werden kann. Zur Gewebeentfernung wird durch das Schneidrohr eine Faßzange in den Körper bzw. die Körperhöhle eingeführt, mit der das zu extrahierende Gewebe gefasst wird. Wird die Zange nun zurückgezogen und das Gewebe gegen die Schneidkante des rotierenden Morcellators gepresst, so kann durch dosierten Zug und gegebenenfalls Variation der Drehgeschwindigkeit und Drehrichtung ein zylinderförmiger Gewebeblock ausgeschnitten und durch das Rohr entfernt werden. Selbst große Gewebemengen können so innerhalb weniger Minuten extrahiert werden.

US5618296 offenbart eine Vorrichtung gemäß dem Oberbegriff des Anspruchs 1. Bei dieser bekannten Vorrichtung ist der Motor als getriebelos auf dem Schneidrohr angeordneter Hohlwellenmotor ausgebildet. Nachteilig bei dieser bekannten Konstruktion ist, dass durch das Aufsetzen des Motors auf das Schneidrohr zwar die Verluste bei der Leistungsübertragung reduziert werden konnten, dafür aber die Demontage zu Reinigungs- und Wartungszwecken deutlich erschwert wurde.

Weitere medizinische Instrumente zum Schneiden von biologischem und insbesondere menschlichem Gewebe sind beispielsweise aus der EP-B1-0 555 803 und der EP-A1-0 806 183 bekannt. Bei diesen bekannten Morcellatoren erfolgt der rotierende Antrieb des Schneidrohres über ein zwischen Motor und Schneidrohr zwischengeschaltetes Zahnrad- und/oder Schneckengetriebe. Die Verwendung dieser der Drehmomentübertragung dienenden Getriebe erzeugen im Betrieb jedoch hohe Leistungsverluste der eigentlichen Motorleistung. Darüber hinaus sind die Getriebe teilweise sehr kompliziert aufgebaut und somit auch sehr teuer.

Aus der EP-A2-0 769 278 ist darüber hinaus ein modulares Trokarsystem bekannt, welches zum Abdichten des hohlen zentralen Kanals des Morcellatorschaftes ein in dem hohlen Kanal angeordnetes Klappenventil aufweist. Nachteilig bei dieser bekannten Konstruktion ist, dass die Klappenflügel des Klappenventils beim Einführen des Schneidrohres in den hohlen zentralen Kanal durch die Schneidkante des Schneidrohres beschädigt werden können.

Davon ausgehend liegt der Erfindung die **Aufgabe** zugrunde, ein medizinisches Instrument der eingangs genannten Art so zu verbessern, dass der Antrieb des Schneidrohres einfach und platzsparend aufgebaut ist und mit geringen Leistungsverlusten erfolgt.

Die **Lösung** dieser Aufgabenstellung ist erfindungsgemäß dadurch gekennzeichnet, dass das Schneidrohr und der Hohlwellenmotor über in Axialrichtung des Schneidrohres verlaufende, am Schneidrohr und/oder am Hohlwellenmotor angeordnete Stifte miteinander gekoppelt sind, die in korrespondierende Ausnehmungen im jeweils anderen Bauteil einsteckbar sind.

Diese Stifte ermöglichen zusammen mit den entsprechenden Ausnehmungen auf einfache Weise eine form- und kraftschlüssige Kopplung von Antrieb und Schneidrohr.

Um einen gleichmäßigen Antrieb des Schneidrohres zu ermöglichen, wird vorgeschlagen, dass das am Schneidrohr vier gleichmäßig um die Längsachse des Schneidrohres verteilt angeordnete, in Axialrichtung des Schneidrohres verlaufende Stifte angeordnet sind. Jedoch sind auch Ausführungen mit weniger, insbesondere drei Stiften, oder mit mehr als vier Stiften möglich.

Die Montage der Kopplung der beiden Bauteile kann erfindungsgemäß dadurch vereinfacht werden, dass am Hohlwellenmotor mehr als vier Ausnehmungen zur Aufnahme der Stifte ausgebildet sind, damit die Bauteile in verschiedenen Positionen zueinander miteinander koppelbar sind.

Weiterhin wird mit der Erfindung vorgeschlagen, dass die das Schneidrohr und den Hohlwellenmotor miteinander koppelnden Stifte an einem auf dem Schneidrohr festlegbaren Adapter angeordnet sind.

Durch die Verwendung des auf dem Schneidrohr angeordneten Hohlwellenmotors, wie dieser beispielsweise für Lüfterantriebe aus der Klimatechnik bekannt ist, ist es möglich den Antrieb äußerst platzsparend auszugestalten. Gemäß einer bevorzugten Ausführungsform der Erfindung treibt der Hohlwellenmotor das Schneidrohr getriebelos direkt an, so dass auch die Leistungsverluste des Antriebsmotors deutlich reduziert werden können. Darüber hinaus vereinfacht die getriebelose Ausführung des Antriebs die Herstellung und Montage erheblich.

Gemäß einer zweiten praktischen Ausführungsform der Erfindung wird vorgeschlagen, dass der Hohlwellenmotor als Außenläufer-Motor ausgebildet ist. Der Außenläufer-Motor zeichnet sich dadurch aus, dass der Rotor des Motors nicht wie bei anderen Elektromaschinen üblich innenliegend angeordnet ist, sondern außen, das heißt den Stator umgebend angeordnet ist.

Die erfindungsgemäße Verwendung des Außenläufer-Motors ist besonders vorteilhaft, da es möglich ist den Motor so auszubilden, dass der Rotor des Außenläufer-Motors das Schneidrohr koaxial umgibt und das Schneidrohr den Stator des Außenläufer-Motors bildet. Die Ausbildung des Schneidrohres als Teil des Schneidrohrantriebes ermöglicht eine besonders kompakte Bauweise bei geringsten Leistungsverlusten der Motorleistung. Um zu bewirken, dass das eigentlich den Stator des Außenläufer-Motors bildende Schneidrohr sich um seine Längsachse drehend angetrieben wird, wird erfindungsgemäß vorgeschlagen, dass im Betrieb der die Handhabe haltende Benutzer derart eine Drehmomentstütze für den Außenläufer-Motor bildet, dass nunmehr das drehbar gelagerte Schneidrohr den Rotor des Außenläufer-Motors bildet.

Die Ausgestaltung des Schneidrohrantriebs mit dem Schneidrohr als Stator des Außenläufer-Motors ist gemäß einer praktischen Ausgestaltungsform der Erfindung dadurch erzielbar, dass das Schneidrohr aus einem magnetisierbaren Material besteht und die Wicklung des Außenläufer-Motors das Schneidrohr koaxial umgebend angeordnet ist.

Schließlich wird mit der Erfindung vorgeschlagen, dass das Schneidrohr in der Handhabe über mindestens ein Lager, insbesondere ein Kugellager, gelagert ist, um eine reibungsarme Drehung des Schneidrohres zu garantieren. Da durch das Andrücken des Schneidrohres gegen das zu morcellierende Gewebe das Schneidrohr auch mit axial wirkenden Kräften beaufschlagt wird, wird erfindungsgemäß weiterhin vorgeschlagen, dass mindestens ein Lager als axiale Kräfte aufnehmendes Schrägkugellager oder Kegelrollenlager ausgebildet ist.

Um einerseits das Einführen des Schneidrohres in das Operationsgebiet zu ermöglichen und andererseits nicht zu behandelndes Gewebe vor der Schneide zu schützen ist gemäß einer bevorzugten Ausführungsform der Erfindung das Schneidrohr distalseitig in einer das Schneidrohr koaxial umgebenden Trokarhülse angeordnet.

Damit es möglich ist, während der Operation mindestens ein weiteres medizinisches Instrument, beispielsweise eine Greifzange zum Ergreifen von zu morcellierendem Gewebe, in das hohle Schneidrohr einsetzen zu können, weist das Schneidrohr eine zentrale Durchgangsbohrung auf.

Das Führen des erfindungsgemäßen Instruments kann für den Operateur dadurch erleichtert werden, dass an der Handhabe ein zusätzlicher, abgewinkelter Handgriff festlegbar ist. Vorzugsweise ist dieser zusätzliche Handgriff um 90° gegenüber der Instrumentenlängsachse abgewinkelt und gewichtsoptimiert ausgebildet.

Weitere Merkmale und Vorteile der Erfindung ergeben sich anhand der zugehörigen Zeichnung, in der verschiedene Ausführungsbeispiele eines erfindungsgemäßen medizinischen Instruments zum Schneiden von biologischem und insbesondere menschlichem Gewebe nur beispielhaft dargestellt sind. In der Zeichnung zeigt:
- Fig. 1: einen ausschnittweisen schematischen Längsschnitt durch eine erste Ausführungsform eines erfindungsgemäßen medizinischen Instruments;
- Fig. 2: eine vergrößerte Detailansicht einer erfindungsgemäßen Kopplung zwischen Schneidrohr und Motor gemäß einer zweiten Ausführungsform der Erfindung;
- Fig. 3: eine perspektivische Explosionszeichnung einer ersten praktischen Ausführungsform eines erfindungsgemäßen Instruments;
- Fig. 4: eine Seitenansicht des Instruments gemäß Fig. 3 im zusammengebauten Zustand;
- Fig. 5: einen Längsschnitt durch das Instrument gemäß Fig. 4;
- Fig. 6: eine Seitenansicht gemäß Fig. 4, jedoch das Instrument mit eingesetztem zusätzlichem Instrument darstellend;
- Fig. 7: eine perspektivische Explosionszeichnung einer zweiten praktischen Ausführungsform eines erfindungsgemäßen Instruments;
- Fig. 8: einen schematischen Längsschnitt durch ein Klappenventil in der geschlossenen Stellung;
- Fig. 9: einen Fig. 8 entsprechenden Längsschnitt, jedoch das Klappenventil in der geöffneten Stellung mit eingesetztem Schneidrohr darstellend und
- Fig. 10: eine schematische Draufsicht auf eine Dichtungskappe.

Das in der Abbildung dargestellte, als Morcellator ausgebildete medizinische Instrument zum Schneiden von biologischem und insbesondere menschlichem Gewebe besteht im wesentlichen aus einem in einer Handhabe 1 drehbar gelagerten hohlen Schneidrohr 2 sowie einem Motor 3, über den das Schneidrohr 2 um seine Längsachse 4 drehbar antreibbar ist. Zum Schneiden des Gewebes weist das Schneidrohr 2 am distalseitigen Ende eine Schneide 2a auf. Die Handhabe 1 wird bei der dargestellten Ausführungsform durch ein Motorgehäuse 3a des Motors 3 gebildet.

Wie weiterhin aus der Abbildung ersichtlich, ist das Schneidrohr 2 bei der dargestellten Ausführungsform zumindest in seinem Bereich distalseitig der Handhabe 1 koaxial von einem beispielsweise als Trokarhülse 5 ausgebildeten Außenrohr umgeben, deren distales Ende unter Ausbildung einer Spitze 5a in Längsrichtung schräg verlaufend ausgebildet ist, wobei die Trokarhülse 5 das Schneidrohr 2 zumindest im Bereich der Spitze 5a distalseitig überragt. Neben dem Offenhalten des Zugangs zum Operationsgebiet dient die Trokarhülse 5 dazu, die Schneide 2a des Schneidrohres 2 beispielsweise beim Einführen des Morcellators in das Operationsgebiet zumindest teilweise abdecken zu können, um keine unbeabsichtigten Verletzungen an zu schonendem Gewebe zu verursachen. Vorteilhafterweise ist die Trokarhülse 5 so ausgebildet, dass sie zwischen einer die Schneide 2a verdeckenden und eine die Schneide 2a freigebenden Stellung in Richtung der Längsachse 4 des Schneidrohres 2 verlagerbar ist.

Selbstverständlich ist es auch möglich, das Schneidrohr 2 so auszubilden, dass es zwischen einer die Spitze 5a der Trokarhülse 5 überragenden und einer hinter die Spitze 5a der Trokarhülse 5 zurückgezogenen Stellung in Richtung der Längsachse 4 des Instruments verlagerbar ist.

Neben der dargestellten Verwendung einer mit einer Spitze 5a versehenen schrägen oder spitzen Trokarhülse 5 ist es selbstverständlich auch möglich, einen wie voranstehend beschriebenen Morcellator mit einer geraden oder stumpfen Trokarhülse 5 zu versehen, die distalseitig einen geraden Abschluss aufweist.

Um das Schneidrohr 2 möglichst reibungsarm drehbar in der Handhabe 1 (dem Motorgehäuse 3a) zu lagern, sind bei dieser Ausführungsform zwei als Kugellager ausgebildete Lager 6 vorgesehen, von denen zumindest eines vorzugsweise als Schrägkugellager oder Kegelrollenlager ausgebildet ist, um auch in axialer Richtung wirkende Kräfte aufnehmen zu können.

Der Motor 3 zum rotierenden Antrieb des Schneidrohres 2 ist als im Bereich des als Handhabe 1 ausgebildeten Motorgehäuses 3a auf dem Schneidrohr 2 angeordneter Hohlwellenmotor ausgebildet, über den das Schneidrohr 2 direkt, das heißt getriebelos antreibbar ist.

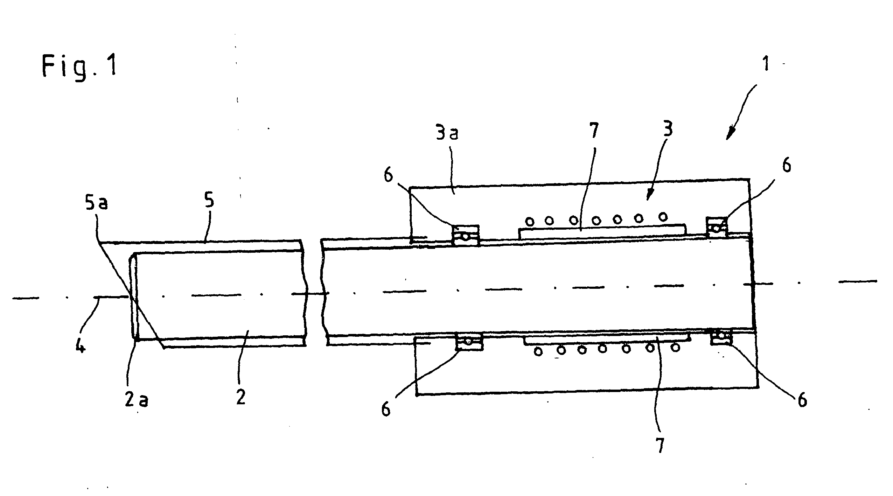

Beim in Fig. 1 dargestellten Ausführungsbeispiel ist der Hohlwellenmotor als Außenläufer-Motor ausgebildet, wobei ein mit der Wicklung versehener Rotor 7 das als Stator des Motors 3 dienende aus einem magnetisierbaren Material hergestellte Schneidrohr 2 koaxial umgibt.

Beim Betrieb eines solchermaßen ausgebildeten Morcellators bildet der mit einer Hand die Handhabe 1 (das Motorgehäuse 3a) festhaltende Bediener des Morcellators die Drehmomentstütze für den Motor 3. Da der Rotor 7 in der Handhabe 1 angeordnet ist, bewirkt dieses Festhalten des Rotors 7 des Außenläufer-Motors, dass nunmehr das Schneidrohr 2 den rotierenden Teil des Motors 3 bildet und sich um seine Längsachse 4 dreht.

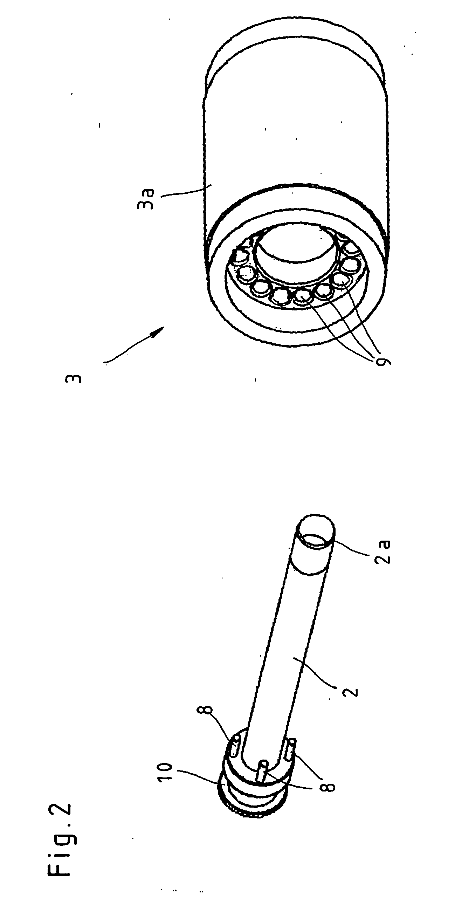

Fig. 2 zeigt eine alternative Ausführungsform des Schneidrohrantriebs. Bei dieser Ausgestaltungsform ist der Motor 3 wiederum als auf dem Schneidrohr 2 angeordneter Hohlwellenmotor ausgebildet, jedoch sind der Motor 3 und das Schneidrohr 2 über in Axialrichtung des Schneidrohres 2 verlaufende, am Schneidrohr 2 angeordnete Stifte 8 miteinander form- und kraftschlüssig miteinander gekoppelt, die in entsprechende Ausnehmungen 9 am Motor 3 einsteckbar sind. Wie weiterhin aus Fig. 2 ersichtlich, sind die Stifte 8 an einem auf dem Schneidrohr 2 angeordneten Adapter 10 angeordnet.

Um eine gleichmäßige und im wesentliche unwuchtfreie Kopplung zu ermöglichen, sind am Schneidrohr 2 bzw. am Adapter 10 vier gleichmäßig um die Längsachse des Schneidrohres 2 verteilt angeordnete Stifte 8 angeordnet. Die Montage der Kopplung der beiden Bauteile 2 und 3 wird dadurch vereinfacht werden, dass am Motor 3 mehr als vier Ausnehmungen 9 zur Aufnahme der Stifte 8 ausgebildet sind, damit die Bauteile 2 und 3 in verschiedenen Positionen zueinander miteinander koppelbar sind.

Selbstverständlich ist es auch möglich, die Kopplung zwischen dem Motor 3 und dem Schneidrohr 2 so auszugestalten, dass die Stifte 8 am Motor 3 und die Ausnehmungen 9 zur Aufnahme der Stifte 8 am Schneidrohr 2 oder dem auf dem Schneidrohr 2 festgelegten Adapter 10 angeordnet sind. Ebenso sind auch Ausführungen mit weniger, insbesondere drei Stiften 8, oder mit mehr als vier Stiften 8 möglich.

Die Verwendung des direkt auf dem Schneidrohr 2 angeordneten Hohlwellenmotors stellt eine besonders platzsparende und einfach zu herzustellende und zu montierende Form des Schneidrohrantriebs dar. Darüber hinaus können durch den getriebelosen direkten Antrieb des Schneidrohres 2 die Leistungsverluste bei der Übertragung des Motordrehmoments auf das Schneidrohr 2 weitestgehend minimiert werden.

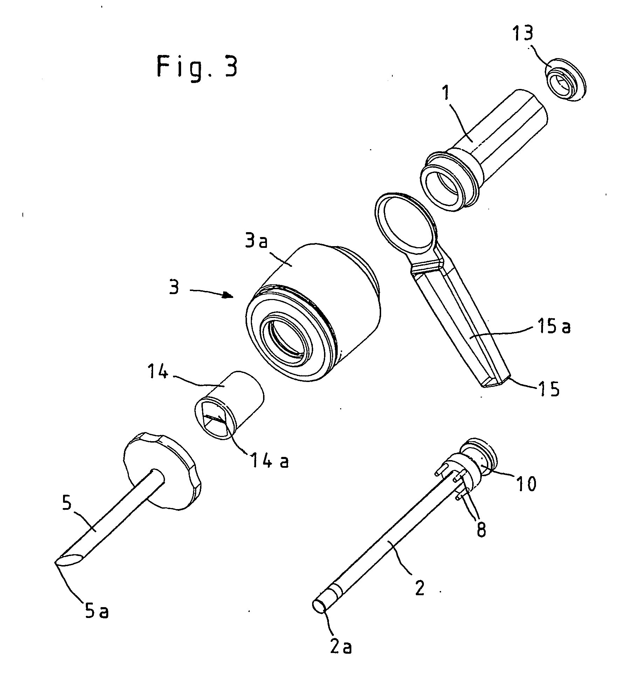

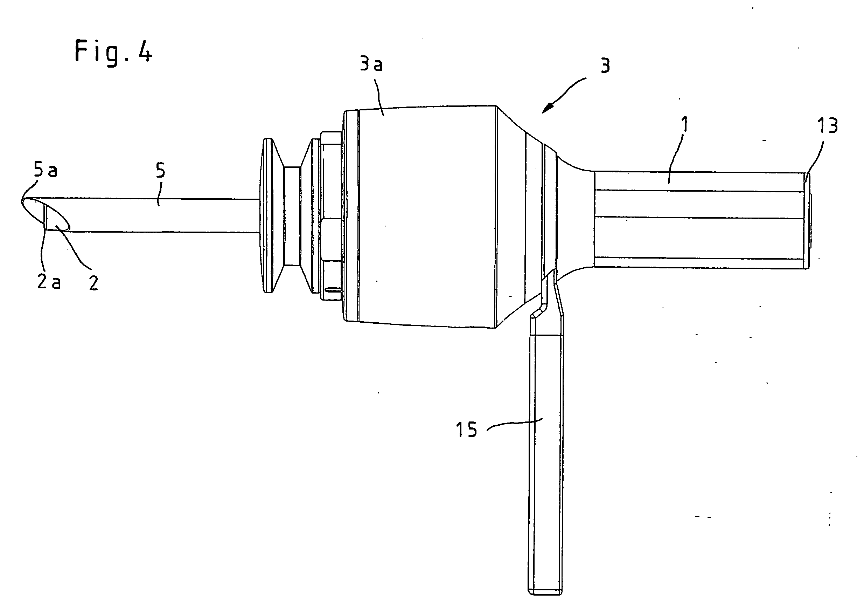

In den Abbildungen Fig. 3 bis Fig. 6 ist eine erste praktische Ausführungsform zur Ausbildung eines als Morcellator für laparoskopische Eingriffe ausgebildeten medizinischen Instruments zum Schneiden von biologischem und insbesondere menschlichem Gewebe. Wie insbesondere aus der Explosionsdarstellung gemäß Fig. 3 ersichtlich, besteht der dargestellte Morcellator im wesentlichen aus einem gleichzeitig das Morcellatorgehäuse bildenden Motorgehäuse 3a des als Hohlwellenmotor ausgebildeten Motors 3, der proximalseitig am Motorgehäuse 3a angeordneten Handhabe 1, der distalseitigen Trokarhülse 5 sowie dem im Inneren des Motorgehäuses 3a sowie der Trokarhülse 5 gelagerten Schneidrohr 2.

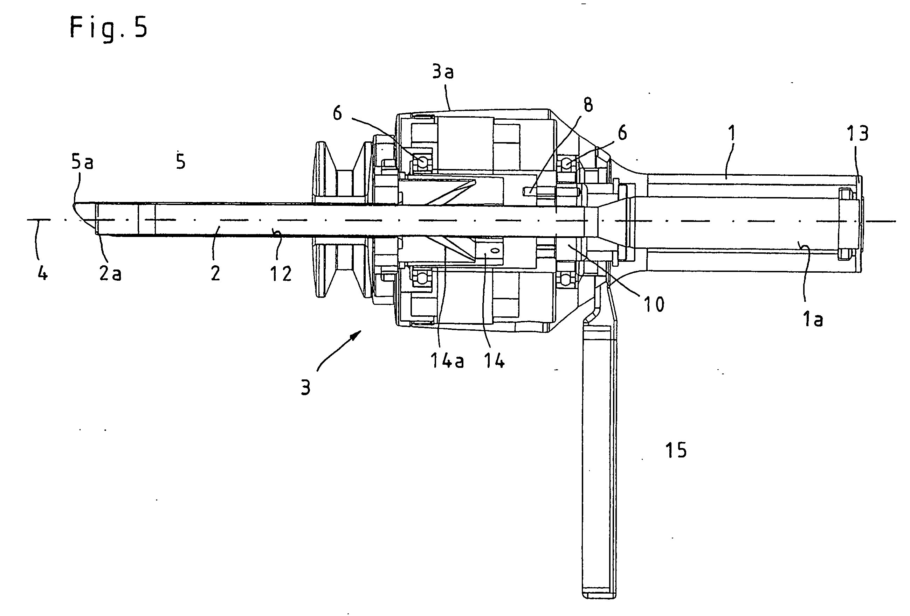

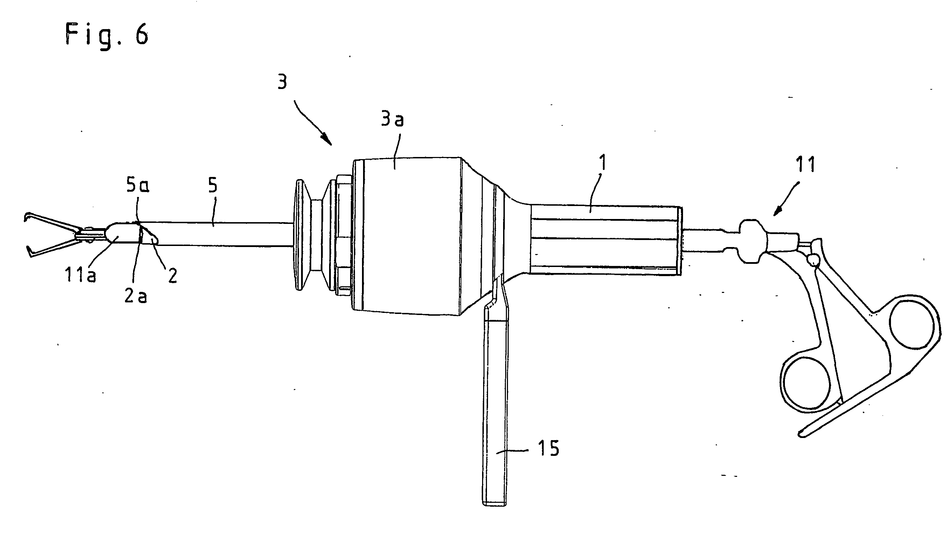

Zur Aufnahme eines weiteren medizinischen Instruments 11, wie dies in Fig. 6 dargestellt ist, weist der Morcellator einen sich entlang der Längsachse 4 des Morcellators erstreckenden zentralen Kanal 12 auf, zu dessen Ausbildung die Handhabe 1 eine zentrale Durchgangsbohrung 1a aufweist. Dieser zentrale Kanal 12 ist insbesondere der Schnittdarstellung gemäß Fig. 5 zu entnehmen.

Da es bei endoskopischen Operationen des Bauchraums üblich ist, den Bauchraum zur Erweiterung des Operationsraums und Ausbildung eines Pneumoperitoneums mit Gas zu befüllen, ist der hohle zentrale Kanal 12 über mindestens ein Dichtungsmittel verschließbar, damit das Gas nicht beispielsweise über die Trokarhülse 5 und den zentralen Kanal 12 aus dem Bauchraum entweichen kann.

Bei der in den Abbildungen Fig. 3 bis Fig. 6 dargestellten Ausführungsform sind zur gasdichten Abdichtung des zentralen Kanals 12 zwei Dichtungsmittel vorgesehen, nämlich einerseits eine am proximalen Ende des zentralen Kanals 12 festlegbare Dichtungskappe 13 sowie ein in den zentralen Kanal 12 einsetzbares Klappenventil 14. Beide Dichtungsmittel 13 und 14 sind unabhängig voneinander und in Kombination miteinander verwendbar.

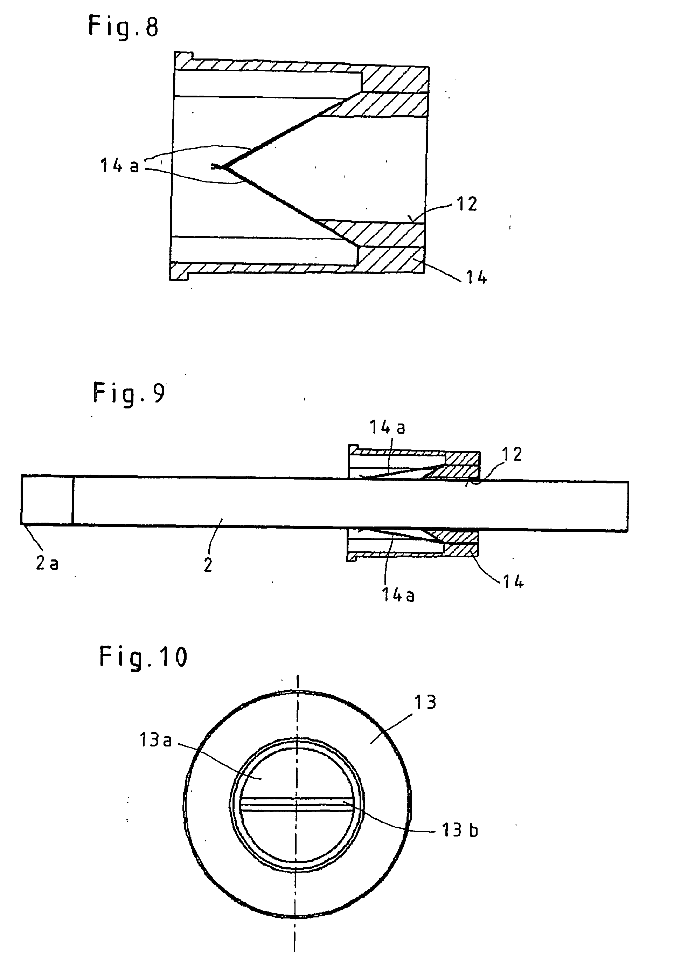

Der Aufbau und die Arbeitsweise des Klappenventils 14 sind insbesondere den schematischen Schnittdarstellungen gemäß Fig. 8 und 9 sowie dem Längsschnitt gemäß Fig. 5 zu entnehmen. Wie aus der Darstellung gemäß Fig. 8 ersichtlich, weist das Klappenventil 14 zwei zum distalen Ende des zentralen Kanals 12 weisende, Klappenflügel 14a auf, die in der geschlossenen Stellung (Fig. 8), also bei nicht in den zentralen Kanal 12 eingesetztem bzw. daraus herausgezogenem Schneidrohr 2, den zentralen Kanal 12 abdichtend fest aneinander anliegen.

Um zu verhindern, dass beim Durchschieben des Schneidrohres 2 durch den zentralen Kanal 12 des Klappenventils 14, wie dies in Fig. 9 dargestellt ist, die Schneide 2a des Schneidrohres 2 mit der scharfen vorderen Schneidkante gegen die Klappenflügel 14a stößt und diese beschädigt, ist der Winkel, unter dem die beiden Klappenflügel 14a des Klappenventils 14 aneinander anliegen, flacher ausgebildet als der Winkel der Schneide 2a am distalen Ende des Schneidrohres 2. Aufgrund der Wahl dieses Winkels der schnabelförmig aneinander anliegenden Klappenflügel 14a wird gewährleistet, dass beim Einführen des Schneidrohres 2 in das Klappenventil 14 die proximalen Enden der Schneidenabschrägung der Schneide 2a des Schneidrohres 2 die Klappenflügel 14a vorrangig in radialer Richtung nach außen öffnen. Schnabelförmig Ausbildung der Klappenflügel 14a bedeutet hierbei, dass die distalen, aneinander anliegenden Enden der Klappenflügel 14a von der Instrumentenachse 4 weg nach außen gekrümmt sind und im Bereich dieser Krümmungen aneinander anliegen.

Bei der in Fig. 9 dargestellten Offenstellung des Klappenventils 14 dichtet das in den zentralen Kanal 12 eingesetzte Schneidrohr 2 den zentralen Kanal 12 ab, da, zumindest im Bereich des Klappenventils 14, der Außendurchmesser des Schneidrohres 2 im wesentlichen dem Innendurchmesser des zentralen Kanals 12 entspricht.

Eine weitere Möglichkeit den zentralen Kanal 12 im Bereich des Schneidrohres 2 gasdicht abzudichten besteht darin, im Bereich des distalen Endes des in das Schneidrohr 2 eingesetzten weiteren medizinischen Instruments 11 einen verdickten Bereich 11a an dem eingesetzten Instrument 11 auszubilden, der dem Innendurchmesser des Schneidrohres 2, insbesondere im Bereich der Schneide 2a entspricht, wie dies der Abbildung Fig. 6 zu entnehmen ist.

Neben der Dichtwirkung des verdickten Bereichs 11a ist dieser verdickte Bereich 11a des zusätzlichen medizinischen Instruments 11 als Schneidkantenschutz verwendbar, der beim Einführen des Schneidrohres 2 in ein Operationsgebiet radial bündig mit der Schneidkante der Schneide 2a in Anlage bringbar ist und in axialer Richtung zumindest bündig mit der Schneidkante abschließt oder distalseitig über die Schneidkante 2a hervorsteht, um das Verletzen von zu schonendem Gewebe auszuschließen.

Die Dichtungskappe 13 zum Verschließen des proximalen Endes des zentralen Kanals 12 ist vorteilhafterweise als aus einem autoklavierbaren Material bestehendes Einmal-Ventil ausgebildet, dessen Dichtungsmaterial 13a beim Einschieben des weiteren medizinischen Instruments 11 in den zentralen Kanal 12 abdichtend am Schaft des eingesetzten Instruments 11 anliegt. Bei der in Fig. 10 dargestellten Ausführungsform der Dichtungskappe 13 ist in dem Dichtungsmaterial 13a ein Schlitz 13b ausgebildet, der durch das Einschieben des Instruments 11 aufgeweitet wird.

Gemäß einer nicht dargestellten alternativen Ausführungsform der Dichtungskappe 13 ist das Dichtungsmaterial 13a als von dem in den zentralen Kanal 12 einsetzbaren Instrument 11 durchstoßbare Membran ausgebildet.

Wie weiterhin aus den Abbildungen Fig. 3, 4 und 6 ersichtlich, ist an der Handhabe 1 ein zusätzlicher, abgewinkelter Handgriff 15 festgelegt, um das Führen des Morcellators für den Operateur zu erleichtern. Vorzugsweise ist dieser zusätzliche Handgriff 15, wie dargestellt, um 90° gegenüber der Instrumentenlängsachse 4 abgewinkelt. Selbstverständlich sind je nach Anwendungsgebiet und/oder Wunsch des Operateurs und/oder ergonomischen Gesichtspunkten auch andere Winkelstellungen des zusätzlichen Handgriffs 15 möglich.

Insbesondere bei der Herstellung des zusätzlichen Handgriffs 15 aus Metall ist es zur Verringerung des Gesamtgewichts des Instruments wichtig, den Handgriff 15 gewichtsoptimiert auszubilden. Hierzu ist es beispielsweise möglich, den Handgriff 15 so auszugestalten, dass dieser im mittleren Bereich 15a einen vollständigen Durchbruch oder aber, wie in Fig. 3 dargestellt, einen Rücksprung aufweist.

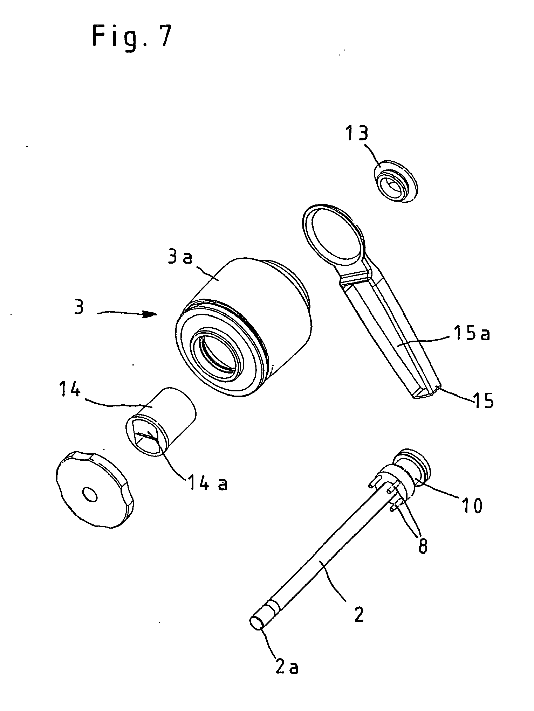

In Fig. 7 ist eine zweite praktische Ausführungsform zur Ausbildung eines als Morcellator ausgebildeten medizinischen Instruments zum Schneiden von biologischem und insbesondere menschlichem Gewebe dargestellt. Dieser für suprazervikale Eingriffe dienende Morcellator unterscheidet sich von dem zuvor insbesondere anhand der Abbildungen Fig. 3, 4 und 6 beschriebenen Morcellator für laparoskopische Eingriffe dadurch, dass dieser keine das Schneidrohr 2 distalseitig umgebende Trokarhülse 5 sowie keine besondere Handhabe 1 aufweist. Statt dessen dient, wie bereits zur Abbildung Fig. 1 beschrieben, das Motorgehäuse 3a als Handhabe 1.

### Bezugszeichenliste

- 1: Handhabe
- 1a: Durchgangsbohrung
- 2: Schneidrohr
- 2a: Schneide
- 3: Motor
- 3a: Motorgehäuse
- 4: Längsachse
- 5: Trokarhülse
- 5a: Spitze
- 6: Lager
- 7: Rotor
- 8: Stift
- 9: Ausnehmung
- 10: Adapter
- 11: medizinisches Instrument
- 11 a: verdickter Bereich
- 12: zentraler Kanal
- 13: Dichtungskappe
- 13a: Dichtungsmaterial
- 13b: Schlitz
- 14: Klappenventil
- 14a: Klappenflügel
- 15: Handgriff
- 15a: mittlerer Bereich

## Patentansprüche

1. Medizinisches Instrument zum Schneiden von biologischem und insbesondere menschlichem Gewebe mit einem über einen Motor (3) um seine Längsachse (4) drehbaren hohlen Schneidrohr (2), an dessen distalem Ende mindestens eine Schneide (2a) angeordnet ist, sowie mit einer Handhabe (1), in der das Schneidrohr (2) führend gelagert ist, wobei der Motor (3) als auf dem Schneidrohr (2) angeordneter Hohlwellenmotor ausgebildet ist,
**dadurch gekennzeichnet,**
**dass** das Schneidrohr (2) und der Hohlwellenmotor über in Axialrichtung des Schneidrohres (2) verlaufende, am Schneidrohr (2) und/oder am Hohlwellenmotor angeordnete Stifte (8) miteinander gekoppelt sind, die in korrespondierende Ausnehmungen (9) im jeweils anderen Bauteil einsteckbar sind.

2. Medizinisches Instrument nach Anspruch 1, **dadurch gekennzeichnet, dass** das am Schneidrohr (2) vier gleichmäßig um die Längsachse des Schneidrohres (2) verteilt angeordnete, in Axialrichtung des Schneidrohres (2) verlaufende Stifte (8) angeordnet sind und am Hohlwellenmotor mindestens vier Ausnehmungen (9) zur Aufnahme der Stifte ausgebildet sind.

3. Medizinisches Instrument nach Anspruch 1 oder 2, **dadurch gekennzeichnet, dass** die das Schneidrohr (2) und den Hohlwellenmotor miteinander koppelnden Stifte (8) an einem auf dem Schneidrohr (2) festlegbaren Adapter (10) angeordnet sind.

4. Medizinisches Instrument nach mindestens einem der Anspruch 1 bis 3, **dadurch gekennzeichnet, dass** das Schneidrohr (2) über den Hohlwellenmotor getriebelos direkt antreibbar ist.

5. Medizinisches Instrument nach Anspruch 1 oder 2, **dadurch gekennzeichnet, dass** der Hohlwellenmotor als Außenläufer-Motor ausgebildet ist.

6. Medizinisches Instrument nach Anspruch 5, **dadurch gekennzeichnet, dass** der Rotor (7) des Außenläufer-Motors das Schneidrohr (2) koaxial umgibt und das Schneidrohr (2) den Stator des Außenläufer-Motors bildet.

7. Medizinisches Instrument nach Anspruch 6, **dadurch gekennzeichnet, dass** im Betrieb der das Motorgehäuse (3a) haltende Benutzer derart eine Drehmomentstütze für den Außenläufer-Motor bildet, dass das drehbar gelagerte Schneirohr (2) den Rotor des Außenläufer-Motors bildet.

8. Medizinisches Instrument nach Anspruch 6 oder 7, **dadurch gekennzeichnet, dass** das Schneidrohr (2) aus einem magnetisierbaren Material besteht und die Wicklung des Außenläufer-Motors das Schneidrohr (2) koaxial umgibt.

9. Medizinisches Instrument nach mindestens einem der Ansprüche 1 bis 8, **dadurch gekennzeichnet, dass** das Schneidrohr (2) im Motorgehäuse über mindestens ein Lager (6), insbesondere ein Kugellager, gelagert ist.

10. Medizinisches Instrument nach Anspruch 9, **dadurch gekennzeichnet, dass** mindestens ein Lager (6) als axiale Kräfte aufnehmendes Schrägkugellager oder Kegelrollenlager ausgebildet ist.

11. Medizinisches Instrument nach mindestens einem der Ansprüche 1 bis 10, **dadurch gekennzeichnet, dass** das Schneidrohr (2) distalseitig in einer das Schneidrohr (2) koaxial umgebenden Trokarhülse (5) angeordnet ist.

12. Medizinisches Instrument nach mindestens einem der Ansprüche 1 bis 11, **dadurch gekennzeichnet, dass** die Handhabe (1) eine zentrale Durchgangsbohrung (1a) aufweist, die proximalseitig eine Verlängerung des hohlen Schneidrohres (2) bildet.

13. Medizinisches Instrument nach mindestens einem der Ansprüche 1 bis 12, **dadurch gekennzeichnet, dass** an der Handhabe (1) ein zusätzlicher, abgewinkelter Handgriff (15) festlegbar ist.

## Claims

1. Medical instrument for cutting biological, particularly human, tissue with a hollow cutting tube (2) which can be rotated about its longitudinal axis (4) by means of a motor (3) and which has at least one blade (2a) at its distal end and with a grip (1) through which the cutting tube (2) is guided, the motor (3) being formed as a hollow-shaft motor sited on the cutting tube (2),
**characterized**
**in that** the cutting tube (2) and the hollow-shaft motor are connected to each other via pins (8) which run in the same direction as the axis of the cutting tube (2), which are sited on the cutting tube (2) and/or on the hollow-shaft motor, and which can be inserted in corresponding recesses (9) in the other component.

2. Medical instrument according to Claim 1, **characterized in that** on the cutting tube (2) there are four pins (8) which are evenly spaced around the longitudinal axis of the cutting tube (2) and which run in the same direction as the axis of the cutting tube (2) and **in that** on the hollow-shaft motor there are at least four recesses (9) to accommodate the pins.

3. Medical instrument according to Claim 1 or 2, **characterized in that** the pins (8) connecting the cutting tube (2) and the hollow-shaft motor to each other are sited on an adapter (10) which can be secured to the cutting tube (2).

4. Medical instrument according to at least one of Claims 1 to 3, **characterized in that** the cutting tube (2) can be driven directly, gearlessly, by means of the hollow-shaft motor.

5. Medical instrument according to Claim 1 or 2, **characterized in that** the hollow-shaft motor is formed as an external-rotor motor.

6. Medical instrument according to Claim 5, **characterized in that** the rotor (7) of the external-rotor motor surrounds the cutting tube (2) coaxially and **in that** the cutting tube (2) forms the stator of the external-rotor motor.

7. Medical instrument according to Claim 6, **characterized in that** in operation the user holding the motor housing (3a) forms a torque support for the external-rotor motor, such that the rotatably mounted cutting tube (2) forms the rotor of the external-rotor motor.

8. Medical instrument according to Claim 6 or 7, **characterized in that** the cutting tube (2) consists of a magnetizable material and **in that** the coil of the external-rotor motor surrounds the cutting tube (2) coaxially.

9. Medical instrument according to at least one of Claims 1 to 8, **characterized in that** the cutting tube (2) is mounted in the motor housing by means of at least one bearing (6), in particular a ball bearing.

10. Medical instrument according to Claim 9, **characterized in that** at least one bearing (6) is formed as an angular contact ball bearing or a tapered roller bearing that absorbs axial forces.

11. Medical instrument according to at least one of Claims 1 to 10, **characterized in that** the distal portion of the cutting tube (2) lies within a trocar sleeve (5) that surrounds the cutting tube (2) coaxially.

12. Medical instrument according to at least one of Claims 1 to 11, **characterized in that** the grip (1) has a central through-hole (1a) which proximally forms an extension of the hollow cutting tube (2).

13. Medical instrument according to least one of Claims 1 to 12, **characterized in that** an additional handle (15) set at an angle can be attached to the grip (1).

## Revendications

1. Instrument médical destiné à couper des tissus biologiques et en particulier des tissus humains, comportant un tube de coupe (2) creux, qui peut tourner autour de son axe longitudinal (4) par l'intermédiaire d'un moteur (3) et dont l'extrémité distale porte au moins une lame de coupe (2a), et comportant un manche (1), dans lequel est logé de manière guidée le tube de coupe (2), le moteur (3) étant réalisé sous forme de moteur à arbre creux, monté sur le tube de coupe (2),
**caractérisé en ce que** le tube de coupe (2) et le moteur à arbre creux sont couplés l'un à l'autre par l'intermédiaire de tétons (8) orientés dans le sens axial du tube de coupe (2), qui sont agencés sur le tube de coupe (2) et/ou sur le moteur à arbre creux et qui sont destinés à être enfichés dans des évidements (9) correspondants réalisés sur respectivement l'autre partie.

2. Instrument médical selon la revendication 1, **caractérisé en ce que** quatre tétons (8), orientés dans le sens axial du tube de coupe (2), sont agencés sur le tube de coupe (2) en étant répartis uniformément autour de l'axe longitudinal du tube de coupe (2) et au moins quatre évidements (9), destinés à recevoir les tétons, sont réalisés sur le moteur à arbre creux.

3. Instrument médical selon la revendication 1 ou 2, **caractérisé en ce que** les tétons (8), par lesquels le tube de coupe (2) et le moteur à arbre creux sont couplés l'un à l'autre, sont agencés sur un adaptateur (10) apte à être fixé sur le tube de coupe (2).

4. Instrument médical selon l'une quelconque des revendications 1 à 3, **caractérisé en ce que** le tube de coupe (2) peut être actionné directement sans engrenage par le moteur à arbre creux.

5. Instrument médical selon la revendication 1 ou 2, **caractérisé en ce que** le moteur à arbre creux est réalisé sous forme de moteur à induit extérieur.

6. Instrument médical selon la revendication 5, **caractérisé en ce que** le rotor (7) du moteur à induit extérieur entoure coaxialement le tube de coupe (2) et le tube de coupe (2) forme le stator du moteur à induit extérieur.

7. Instrument médical selon la revendication 6, **caractérisé en ce que**, en cours de service, l'utilisateur qui tient le boîtier du moteur (3a) constitue un support de couple de rotation pour le moteur à induit extérieur, de telle sorte que le tube de coupe (2) monté rotatif forme le rotor du moteur à induit extérieur.

8. Instrument médical selon la revendication 6 ou 7, **caractérisé en ce que** le tube de coupe (2) est réalisé dans un matériau magnétisable et l'enroulement du moteur à induit extérieur entoure coaxialement le tube de coupe (2).

9. Instrument médical selon l'une quelconque des revendications 1 à 8, **caractérisé en ce que** le tube de coupe (2) est monté dans le boîtier du moteur par l'intermédiaire d'au moins un palier (6), en particulier un palier à roulements.

10. Instrument médical selon la revendication 9, **caractérisé en ce qu'**au moins un palier (6) est réalisé sous forme de roulement à billes à portée oblique ou sous forme de roulement à galets coniques, destinés à absorber des forces axiales.

11. Instrument médical selon l'une quelconque des revendications 1 à 10, **caractérisé en ce que** le tube de coupe (2) est monté, du côté distal, dans un manchon de trocart (5) entourant coaxialement le tube de coupe (2).

12. Instrument médical selon l'une quelconque des revendications 1 à 11, **caractérisé en ce que** le manche (1) comporte une forure débouchante (1a) centrale qui, du côté proximal, forme un prolongement du tube de coupe (2) creux.

13. Instrument médical selon l'une quelconque des revendications 1 à 12, **caractérisé en ce qu'**un manche (15) supplémentaire coudé peut être fixé contre le manche (1).
